# EUROPEAN PATENT SPECIFICATION

(11) **EP 3 436 495 B1**
(45) Date of publication and mention of the grant of the patent: **22.09.2021**
(21) Application number: 17717033.9
(22) Date of filing: 28.03.2017
(51) Int. Cl.: C08G 18/48, C08G 18/66, C08G 18/72, C08G 18/08, C08G 18/76

(54) **THERMOPLASTIC POLYURETHANE COMPOSITIONS FOR SOLID FREEFORM FABRICATION OF ORAL CARE AND MEDICAL DEVICES AND COMPONENTS**
THERMOPLASTISCHE POLYURETHANZUSAMMENSETZUNGEN FÜR FESTSTOFFFREIFORMHERSTELLUNG VON MUNDPFLEGE- UND MEDIZINPRODUKTEN UND KOMPONENTEN
COMPOSITIONS THERMOPLASTIQUES DE POLYURÉTHANE DESTINÉES À LA FABRICATION D'UNE FORME LIBRE SOLIDE DE DISPOSITIFS DE SOINS BUCCODENTAIRES ET MÉDICAUX ET CONSTITUANTS

(30) Priority: 31.03.2016 US 201662315905 P
(43) Date of publication of application: 06.02.2019
(60) Divisional application: 21161865.7
(73) Proprietor: Lubrizol Advanced Materials, Inc., Cleveland, OH 44141-3247 (US)
(72) Inventor: GREEN, Jennifer, Cleveland, Ohio 44141-3247 (US); COX, John M., Cleveland, Ohio 44141-3247 (US); VONTORCIK, Joseph J., Jr., Cleveland, Ohio 44141-3247 (US); MORGAN, Barbara, Cleveland, Ohio 44141-3247 (US)
(74) Representative: Bradley, Josephine Mary
(86) International application number: PCT/US2017/024503
(87) International publication number: WO 2017/172740

(56) References cited:
- WO-A1-2015/109141
- WO-A1-2015/109143
- WO-A1-2015/197515
- WO-A1-2017/015072
- WO-A1-2017/015073

## Description

### FIELD OF THE INVENTION

The invention relates to compositions and methods for the direct solid freeform fabrication of oral care devices or components and applications. The oral care devices can be formed from biocompatible thermoplastic polyurethanes suited for such processing. The useful thermoplastic polyurethanes are derived from (a) an aromatic diisocyanate component, and (b) a chain extender component; wherein the composition is free of polyol components other than the chain extender component.

### BACKGROUND

Solid Freeform Fabrication (SFF), also referred to as additive manufacturing, is a technology enabling fabrication of arbitrarily shaped structures directly from computer data via additive formation steps. The basic operation of any SFF system consists of slicing a three-dimensional computer model into thin cross sections, translating the result into two-dimensional position data and feeding the data to control equipment which fabricates a three-dimensional structure in a layerwise manner.

Solid freeform fabrication entails many different approaches, including three-dimensional printing, electron beam melting, stereolithography, selective laser sintering, laminated object manufacturing, fused deposition modeling and others.

The differences between these processes lies in the way the layers are placed to create parts, as well as in the materials utilized. Some methods, such as selective laser sintering (SLS), fused deposition modeling (FDM) or fused filament fabrication (FFF), melt or soften the material to produce the layers. Other methods, such as stereolithography (SLA), cure liquid materials.

Typically, additive manufacturing for thermoplastics utilizes two types of printing methods. In the first method, known as an extrusion type, a filament and/or a resin (referred to as "pellet printing") of the subject material is softened or melted then deposited by the machine in layers to form the desired object. Extrusion type methods are known as fused deposition modeling (FDM) or fused filament fabrication (FFF). In extrusion methods, a thermoplastic resin or a strand of thermoplastic filament is supplied to a nozzle head which heats the thermoplastic and turns the flow on and off. The part is constructed by extruding small beads of material which harden to form layers.

The second method is the powder or granular type where a powder is deposited in a granular bed and then fused to the previous layer by selective fusing or melting. The technique typically fuses parts of the layer using a high powered laser. After each cross-section is processed, the powder bed is lowered. A new layer of powdered material is then applied and the steps are repeated until the part is fully constructed. Often, the machine is designed with the capability to preheat the bulk powder bed material to slightly below its melting point. This reduces the amount of energy and time for the laser to increase the temperature of the selected regions to the melting point.

Unlike extrusion methods, the granular or powder methods use the unfused media to support projections or ledges and thin walls in the part being produced. This reduces or eliminates the need for temporary supports as the piece is being constructed. Specific methods include selective laser sintering (SLS), selective heat sintering (SHS) and selective laser melting (SLM). In SLM, the laser completely melts the powder. This allows the formation of a part in a layer-wise method that will have the mechanical properties similar to those of conventionally manufactured parts. Another powder or granular method utilizes an inkjet printing system. In this technique, the piece is created layer-wise by printing a binder in the cross-section of the part using an inkjet-like process on top of a layer of powder. An additional layer of powder is added and the process is repeated until each layer has been printed.

Current solid freeform fabrication for oral care devices and applications has been focused on indirect fabrication, such as printing of molds which are subsequently filled with a material or the printing of a form over which a thermoformed device is then molded; or for medical applications involving visualization, demonstration and mechanical prototyping, e.g., where expected outcomes can be modeled prior to performing procedures based on a 3D-printed prototype. Thus, SFF facilitates rapid fabrication of functioning prototypes with minimal investment in tooling and labor. Such rapid prototyping shortens the product development cycle and improves the design process by providing rapid and effective feedback to the designer. SFF can also be used for rapid fabrication of non-functional parts, e.g., models and the like, for the purpose of assessing various aspects of a design such as aesthetics, fit, assembly and the like.

Current materials utilized in additive manufacturing for oral care and medical applications typically include ABS, nylon, polycarbonates, polycaprolactone, polylactic acid (PLA), poly-L-lactic acid (PLLA) and photopolymers/cured liquid materials. Some of these materials are limited to applications outside the body, such as prototypes, molds, surgical planning and anatomical models, owing to their lack of biocompatibility or long term biodurability.

Given the attractive combination of properties thermoplastic polyurethanes offer, and the wide variety of articles made using more conventional means of fabrication, it would be desirable to identify and/or develop thermoplastic polyurethanes well suited for direct solid freeform fabrication of oral care and medical devices, components and applications.

### SUMMARY

The disclosed technology provides an oral care device or component, including an additive-manufactured thermoplastic polyurethane composition derived from (a) a polyisocyanate component comprising an aromatic diisocyanate; (b) a chain extender component; wherein the composition is free of polyol components other than the chain extender component.

The disclosed technology further provides an oral care device or component in which the chain extender component comprises 1,6-hexanediol (HDO), 1,4-cyclohexane dimethanol (CHDM) or combinations thereof.

The disclosed technology further provides an oral care device or component in which the aromatic diisocyanate includes MDI.

The disclosed technology further provides an oral care device or component in which the additive manufacturing comprises fused deposition modeling or selective laser sintering.

The disclosed technology further provides an oral care device or component in which the thermoplastic polyurethane is biocompatible.

The disclosed technology further provides an oral care device or component in which the chain extender component is from 25 wt% to 35 wt% of the total weight of the composition.

The disclosed technology further provides an oral care device or component in which the polyisocyanate component further includes TDI, IPDI, LDI, BDI, PDI, CHDI, TODI, NDI, HXDI or any combination thereof.

The disclosed technology further provides an oral care device or component in which in which the thermoplastic polyurethane further includes one or more of colorants, antioxidants (including phenolics, phosphites, thioesters and/or amines), radio opacifiers, stabilizers, lubricatns, inhibitors, hydrolysis stabilizers, hindered amine light stabilizers, benzotriazole UV absorber, heat stabilizers, stabilizers to prevent discoloration, dyes, pigments, reinforcing agents, or any combination thereof.

The disclosed technology further provides an oral care device or component in which the oral care device or component includes one or more of a dental aligner, a dental retainer or an orthodontic device.

The disclosed technology further provides an oral care device or component in which the oral care device or component is personalized to a patient.

The disclosed technology further provides a method of fabricating a three-dimensional oral care device or component including the steps of (I) operating a system for solid freeform fabrication of an object in which the system includes a solid freeform fabrication apparatus that operates to form a three-dimensional oral care device or component from a building material including a thermoplastic polyurethane derived from (a) a polyisocyanate component including an aromatic diisocyanate; (b) a chain extender component; wherein the composition is free of polyol components other than the chain extender component.

The disclosed technology further provides a directly formed oral care device or component including a selectively deposited thermoplastic polyurethane composition derived from (a) an an aromatic diisocyanate; and (b) a chain extender component; wherein the composition is free of polyol components other than the chain extender component.

### DETAILED DESCRIPTION

Various preferred features and embodiments will be described below by way of non-limiting illustration.

The disclosed technology provides thermoplastic polyurethane compositions useful for the direct solid freeform fabrication of oral care and medical devices and components. The described thermoplastic polyurethanes are biocompatible and biodurable, as well as being free from processing aids required by conventional materials used for solid freeform fabrication methods of oral care devices and components.

### The Thermoplastic Polyurethanes.

The thermoplastic polyurethanes useful in the described technology are derived from (a) an aromatic diisocyanate component, and (b) a chain extender component; wherein the composition is free of polyol components other than the chain extender component. The TPU compositions described herein are made using (a) a polyisocyanate component. The polyisocyanate and/or polyisocyanate component includes one or more polyisocyanates. In some embodiments, the polyisocyanate component includes one or more diisocyanates.

In some embodiments, the polyisocyanate and/or polyisocyanate component includes an alpha, omega-alkylene diisocyanate having from 5 to 20 carbon atoms.

In some embodiments, the polyisocyanate component includes one or more aromatic diisocyanates. In some embodiments, the polyisocyanate component is essentially free of, or even completely free of, aliphatic diisocyanates.

Examples of useful polyisocyanates include aromatic diisocyanates such as 4,4'-methylenebis(phenyl isocyanate) (MDI), m-xylene diisocyanate (XDI), phenylene-1,4-diisocyanate, naphthalene-1,5-diisocyanate, and toluene diisocyanate (TDI); as well as aliphatic diisocyanates such as isophorone diisocyanate (IPDI), 1,4-cyclohexyl diisocyanate (CHDI), decane-1,10-diisocyanate, lysine diisocyanate (LDI), 1,4-butane diisocyanate (BDI), isophorone diisocyanate (PDI), 3,3'-dimethyl-4,4'-biphenylene diisocyanate (TODI), 1,5-naphthalene diisocyanate (NDI), and dicyclohexylmethane-4,4'-diisocyanate (H12MDI). Mixtures of two or more polyisocyanates may be used. In some embodiments, the polyisocyanate is MDI.

The TPU compositions described herein are made using b) a chain extender component. Chain extenders include aromatic glycols, diols, diamines, and combination thereof.

Suitable chain extenders include relatively small polyhydroxy compounds, for example lower aliphatic or short chain glycols having from 2 to 20, or 2 to 12, or 2 to 10 carbon atoms. Suitable examples include ethylene glycol, diethylene glycol, propylene glycol, dipropylene glycol (DPG), 1,4-butanediol (BDO), 1,6-hexanediol (HDO), 1,3-butanediol, 1,5-pentanediol, neopentylglycol, 1,4-cyclohexanedimethanol (CHDM), 2,2-bis[4-(2-hydroxyethoxy) phenyl]propane (HEPP), hexamethylenediol, heptanediol, nonanediol, dodecanediol, 3-methyl-1,5-pentanediol, ethylenediamine, butanediamine, hexamethylenediamine, and hydroxyethyl resorcinol (HER), and the like, as well as mixtures thereof. In some embodiments the chain extender includes HDO. In some embodiments the chain extender includes HDO and CHDM.

The thermoplastic polyurethane is free from a polyol component, the mole ratio of the chain extender to the polyol is infinite.

The thermoplastic polyurethanes described herein may also be considered to be thermoplastic polyurethane (TPU) compositions. In such embodiments, the compositions may contain one or more TPU. These TPU are prepared by reacting:
a) the polyisocyanate component described above; and the chain extender component described above, where the reaction may be carried out in the presence of a catalyst. At least one of the TPU in the composition must meet the parameters described above making it suitable for solid freeform fabrication, and in particular fused deposition modeling.

The means by which the reaction is carried out is not overly limited, and includes both batch and continuous processing. In some embodiments, the technology deals with batch processing of aromatic TPU. In some embodiments, the technology deals with continuous processing of aromatic TPU.

The described compositions include the TPU materials described above and also TPU compositions that include such TPU materials and one or more additional components. These additional components include other polymeric materials that may be blended with the TPU described herein. These additional components include one or more additives that may be added to the TPU, or blend containing the TPU, to impact the properties of the composition.

The TPU described herein may also be blended with one or more other polymers. The polymers with which the TPU described herein may be blended are not overly limited. In some embodiments, the described compositions include two or more of the described TPU materials. In some embodiments, the compositions include at least one of the described TPU materials and at least one other polymer, which is not one of the described TPU materials.

Polymers that may be used in combination with the TPU materials described herein also include more conventional TPU materials such as non-caprolactone polyester-based TPU, polyether-based TPU, or TPU containing both non-caprolactone polyester and polyether groups. Other suitable materials that may be blended with the TPU materials described herein include polycarbonates, polyolefins, styrenic polymers, acrylic polymers, polyoxymethylene polymers, polyamides, polyphenylene oxides, polyphenylene sulfides, polyvinylchlorides, chlorinated polyvinylchlorides, polylactic acids, or combinations thereof.

Polymers for use in the blends described herein include homopolymers and copolymers. Suitable examples include: (i) a polyolefin (PO), such as polyethylene (PE), polypropylene (PP), polybutene, ethylene propylene rubber (EPR), polyoxyethylene (POE), cyclic olefin copolymer (COC), or combinations thereof; (ii) a styrenic, such as polystyrene (PS), acrylonitrile butadiene styrene (ABS), styrene acrylonitrile (SAN), styrene butadiene rubber (SBR or HIPS), polyalphamethylstyrene, styrene maleic anhydride (SMA), styrene-butadiene copolymer (SBC) (such as styrene-butadiene-styrene copolymer (SBS) and styrene-ethylene/butadiene-styrene copolymer (SEBS)), styrene-ethylene/propylene-styrene copolymer (SEPS), styrene butadiene latex (SBL), SAN modified with ethylene propylene diene monomer (EPDM) and/or acrylic elastomers (for example, PS-SBR copolymers), or combinations thereof; (iii) a thermoplastic polyurethane (TPU) other than those described above; (iv) a polyamide, such as Nylon™, including polyamide 6,6 (PA66), polyamide 1,1 (PA11), polyamide 1,2 (PA12), a copolyamide (COPA), or combinations thereof; (v) an acrylic polymer, such as polymethyl acrylate, polymethylmethacrylate, a methyl methacrylate styrene (MS) copolymer, or combinations thereof; (vi) a polyvinylchloride (PVC), a chlorinated polyvinylchloride (CPVC), or combinations thereof; (vii) a polyoxyemethylene, such as polyacetal; (viii) a polyester, such as polyethylene terephthalate (PET), polybutylene terephthalate (PBT), copolyesters and/or polyester elastomers (COPE) including polyether-ester block copolymers such as glycol modified polyethylene terephthalate (PETG), polylactic acid (PLA), polyglycolic acid (PGA), copolymers of PLA and PGA, or combinations thereof; (ix) a polycarbonate (PC), a polyphenylene sulfide (PPS), a polyphenylene oxide (PPO), or combinations thereof; or combinations thereof.

In some embodiments, these blends include one or more additional polymeric materials selected from groups (i), (iii), (vii), (viii), or some combination thereof. In some embodiments, these blends include one or more additional polymeric materials selected from group (i). In some embodiments, these blends include one or more additional polymeric materials selected from group (iii). In some embodiments, these blends include one or more additional polymeric materials selected from group (vii). In some embodiments, these blends include one or more additional polymeric materials selected from group (viii).

The additional optional additives suitable for use in the TPU compositions described herein are not overly limited. Suitable additives include pigments, UV stabilizers, UV absorbers, antioxidants, lubricity agents, heat stabilizers, hydrolysis stabilizers, cross-linking activators, biocompatible flame retardants, layered silicates, colorants, reinforcing agents, adhesion mediators, impact strength modifiers, antimicrobials, radio opacifiers, fillers and any combination thereof. It is to be noted that the TPU compositions of the invention disclosed herein do not require the use of inorganic, organic or inert fillers, such as are talc, calcium carbonate, TiO2, powders which, while not wishing to be bound by theory, it is believed may assist in printability of the TPU composition. Thus, in some embodiments, the disclosed technology may include a filler, and in some embodiments, the disclosed technology may be free of fillers.

The TPU compositions described herein may also include additional additives, which may be referred to as a stabilizer. The stabilizers may include antioxidants such as phenolics, phosphites, thioesters, and amines, light stabilizers such as hindered amine light stabilizers and benzothiazole UV absorbers, and other process stabilizers and combinations thereof. In one embodiment, the preferred stabilizer is Irganox 1010 from BASF and Naugard 445 from Chemtura. The stabilizer is used in the amount from about 0.1 weight percent to about 5 weight percent, in another embodiment from about 0.1 weight percent to about 3 weight percent, and in another embodiment from about 0.5 weight percent to about 1.5 weight percent of the TPU composition.

Still further optional additives may be used in the TPU compositions described herein. The additives include colorants, antioxidants (including phenolics, phosphites, thioesters, and/or amines), stabilizers, lubricants, inhibitors, hydrolysis stabilizers, light stabilizers, hindered amines light stabilizers, benzotriazole UV absorber, heat stabilizers, stabilizers to prevent discoloration, dyes, pigments, reinforcing agents and combinations thereof.

All of the additives described above may be used in an effective amount customary for these substances. The non-flame retardants additives may be used in amounts of from about 0 to about 30 weight percent, in one embodiment from about 0.1 to about 25 weight percent, and in another embodiment about 0.1 to about 20 weight percent of the total weight of the TPU composition.

These additional additives can be incorporated into the components of, or into the reaction mixture for, the preparation of the TPU resin, or after making the TPU resin. In another process, all the materials can be mixed with the TPU resin and then melted or they can be incorporated directly into the melt of the TPU resin.

The TPU materials described above may be prepared by a process that includes the step of (I) reacting: a) the aromatic diisocyanate component described above; b) the chain extender component described above, where the reaction may be carried out in the presence of a catalyst, resulting in a thermoplastic polyurethane composition.

The process may further include the step of: (II) mixing the TPU composition of step (I) with one or more blend components, including one or more additional TPU materials and/or polymers, including any of those described above.

The process may further include the step of: (II) mixing the TPU composition of step (I) with one or more of the additional additives described above.

The process may further include the step of: (II) mixing the TPU composition of step (I) with one or more blend components, including one or more additional TPU materials and/or polymers, including any of those described above, and/or the step of: (III) mixing the TPU composition of step (I) with one or more of the additional additives described above.

### The Systems and Methods.

The solid freeform fabrication systems and the methods of using the same useful in the described technology are not overly limited. It is noted that the described technology provides certain thermoplastic polyurethanes that are better suited for the solid freeform fabrication of medical devices and components, than current materials and other thermoplastic polyurethanes. It is noted that some solid freeform fabrication systems, including some fused deposition modeling systems may be better suited for processing certain materials, including thermoplastic polyurethanes, due to their equipment configurations, processing parameters, etc. However, the described technology is not focused on the details of solid freeform fabrication systems, including some fused deposition modeling systems, rather the described technology is focused on providing certain thermoplastic polyurethanes that are better suited for solid freeform fabrication of medical devices and components.

The extrusion-type additive manufacturing systems and processes useful in the present invention include systems and processes that build parts layer-by-layer by heating the building material to a semi-liquid state and extruding it according to computer-controlled paths. The material, supplied as a strand or resin, may be dispensed as a semi-continuous flow and/or filament of material from the dispenser or it may alternatively be dispensed as individual droplets. FDM often uses two materials to complete a build. A modeling material is used to constitute the finished piece. A support material may also be used to act as scaffolding for the modeling material. The building material, e.g., TPU, is fed from the systems material stores to its print head, which typically moves in a two dimensional plane, depositing material to complete each layer before the base moves along a third axis to a new level and/or plane and the next layer begins. Once the system is done building, the user may remove the support material away or even dissolve it, leaving a part that is ready to use. In some embodiments, the additive manufacturing systems and processes will include a support material which includes a TPU different from the inventive TPU disclosed herein. In some embodiments, the systems and processes are free of the support material.

The powder or granular type of additive manufacturing systems and processes useful in the present invention SLS involves the use of a high power laser (for example, a carbon dioxide laser to fuse small particles of the material, e.g. TPU, into a mass that has a desired three-dimensional shape. Production by selective fusion of layers is a method for producing articles that consists in depositing layers of materials in powder form, selectively melting a portion or a region of a layer, depositing a new layer of powder and again melting a portion of said layer, and continuing in this manner until the desired object is obtained. The selectivity of the portion of the layer to be melted is obtained for example by using absorbers, inhibitors, masks, or via the input of focused energy, such as a laser or electromagnetic beam, for example. Sintering by the addition of layers is preferred, in particular rapid prototyping by sintering using a laser. Rapid prototyping is a method used to obtain parts of complex shape without tools and without machining, from a three-dimensional image of the article to be produced, by sintering superimposed powder layers using a laser. General information about rapid prototyping by laser sintering is provided in U.S. Pat. No. 6,136,948 and applications WO96/06881 and US20040138363.

Machines for implementing these methods may comprise a construction chamber on a production piston, surrounded on the left and right by two pistons feeding the powder, a laser, and means for spreading the powder, such as a roller. The chamber is generally maintained at constant temperature to avoid deformations.

Other production methods by layer additions' such as those described in WO 01/38061 and EP1015214 are also suitable. These two methods use infrared heating to melt the powder. The selectivity of the molten parts is obtained in the case of the first method by the use of inhibitors, and in the case of the second method by the use of a mask. Another method is described in application DE10311438. In this method, the energy for melting the polymer is supplied by a microwave generator and selectivity is obtained by using a susceptor.

The disclosed technology further provides the use of the described thermoplastic polyurethanes in the described systems and methods, and the medical devices and components made from the same.

### The Medical Devices, Components and Applications.

The processes described herein may utilize the thermoplastic polyurethanes described herein to produce various medical devices and components.

As with all additive manufacturing there is particular value for such technology in making articles as part of rapid prototyping and new product development, as part of making custom and/or one time only parts, or similar applications where mass production of an article in large numbers is not warranted and/or practical.

Useful oral care devices and components which may be additive-manufactured from the compositions of the invention include dental aligners, retainers, orthodontic devices, and the like. Useful medical devices and component which may be additive-manufactured from the compositions of the invention include splints, external support braces, orthopedic devices, and the like.

The amount of each chemical component described is presented exclusive of any solvent or diluent oil, which may be customarily present in the commercial material, that is, on an active chemical basis, unless otherwise indicated. However, unless otherwise indicated, each chemical or composition referred to herein should be interpreted as being a commercial grade material which may contain the isomers, byproducts, derivatives, and other such materials which are normally understood to be present in the commercial grade.

It is known that some of the materials described above may interact in the final formulation, so that the components of the final formulation may be different from those that are initially added. For instance, metal ions (of, e.g., a flame retardant) can migrate to other acidic or anionic sites of other molecules. The products formed thereby, including the products formed upon employing the composition of the technology described herein in its intended use, may not be susceptible of easy description. Nevertheless, all such modifications and reaction products are included within the scope of the technology described herein; the technology described herein encompasses the composition prepared by admixing the components described above.

### EXAMPLES

The technology described herein may be better understood with reference to the following non-limiting examples.

### Example 1

*Materials.* Several thermoplastic polyurethanes (TPU) are prepared and evaluated for their suitability of use in direct solid free form fabrication of an oral care device.

TPU-A (not according to the invention) is a TPU containing a polyether polyol with a molar ratio of chain extender to polyol of about 31.3.

TPU-B (not according to the invention) is a TPU containing an ethylene oxide-capped propylene oxide polyol with a molar ratio of chain extender to polyol of about 178.

TPU-C (not according to the invention) is a TPU containing a polyester polyol with a molar ratio of chain extender to polyol of about 29.7. Inventive TPU-D is a TPU containing no polyol, and thus having an infinite molar ratio of polyol to chain extender.

Each TPU material is tested to determine its suitability for use in select freeform fabrication processes. Each TPU material is extruded from resin into approximately 1.8mm diameter rods using s single screw extruder. Tensile bars are printed utilizing a fused deposition modeling process on a MakerBot 2X desktop 3D printer running MakerBot Desktop Software Version 3.7 with the following test parameters:

| | |
|---|---|
| Extrusion Temperature | 200°C-230°C |
| Build Platform Temperature | 40°C-150°C |
| Print Speed | 30 mm/s - 120 mm/s |

Results of this testing are summarized below in Table 1.

**Table 1**

| | TPU-A | TPU-B | TPU-C | TPU-D |
|---|---|---|---|---|
| Chain Extender:Polyol mole ratio | 31.3 | 178 | 29.7 | ∞ |
| Print Speed (mm/sec) | 90 | 90 | 90 | 90 |

As illustrated by the results, the inventive TPU-D compositions provide compositions which are suitable for solid freeform fabrication.

### Example 2

A 3D-printed sample of inventive TPU composition D was evaluated for tensile strength and elongation on the "x", "y" and "z" axes (identified per ASTM 52921) pursuant to ASTM D412 in comparison with a standard injection-molded TPU material of the same composition. Results are shown in Table 2 below:

**Table 2**

| | Tensile Strength (psi) |
|---|---|
| Standard Injection Molded TPU "D" | 7021 ± 128 |
| TPU-D xy axis | 5951 ± 226 |
| TPU-D xz axis | 8117 ± 538 |
| TPU-D zx axis | 1133 ±519 |

As can be seen in Table 2, the ultimate tensile strength of the inventive 3D printed TPU material is comparable to that of an injection molded material when printed using the xz axis direction.

Molecular weight distributions can be measured on the Waters gel permeation chromatograph (GPC) equipped with Waters Model 515 Pump, Waters Model 717 autosampler and Waters Model 2414 refractive index detector held at 40°C. The GPC conditions may be a temperature of 40°C, a column set of Phenogel Guard + 2x mixed D (5u), 300 x 7.5 mm, a mobile phase of tetrahydrofuran (THF) stabilized with 250 ppm butylated hydroxytoluene, a flow rate of 1.0 ml/min, an injection volume of 50 µl, sample concentration ∼0.12%, and data acquisition using Waters Empower Pro Software. Typically a small amount, typically approximately 0.05 gram of polymer, is dissolved in 20 ml of stabilized HPLC-grade THF, filtered through a 0.45-micron polytetrafluoroethylene disposable filter (Whatman), and injected into the GPC. The molecular weight calibration curve may be established with EasiCal® polystyrene standards from Polymer Laboratories.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of' and "consisting of," where "consisting of' excludes any element or step not specified and "consisting essentially of' permits the inclusion of additional un-recited elements or steps that do not materially affect the basic and novel characteristics of the composition or method under consideration. That is "consisting essentially of' permits the inclusion of substances that do not materially affect the basic and novel characteristics of the composition under consideration.

While certain representative embodiments and details have been shown for the purpose of illustrating the subject technology described herein, it will be apparent to those skilled in this art that various changes and modifications can be made therein without departing from the scope of the subject invention. In this regard, the scope of the technology described herein is to be limited only by the following claims.

## Claims

1. An oral care device or component, comprising:
an additive-manufactured thermoplastic polyurethane composition derived from (a) a polyisocyanate component comprising an aromatic diisocyanate; (b) a chain extender component; wherein the composition is free of polyol components other than the chain extender component.

2. The oral care device or component of claim 1, wherein the chain extender component comprises 1,6-hexanediol (HDO), 1,4-cyclohexane dimethanol (CHDM) or combinations thereof.

3. The oral care device or component claim 1, wherein the aromatic diisocyanate comprises MDI.

4. The oral care device or component of claim 1, wherein the additive manufacturing comprises fused deposition modeling or selective laser sintering.

5. The oral care device or component of claim 1, wherein the thermoplastic polyurethane is biocompatible.

6. The oral care device or component of claim 1, wherein the chain extender component is from 25 wt% to 35 wt% of the total weight of the composition.

7. The oral care device or component of claim 1, wherein the polyisocyanate component further comprise TDI, IPDI, LDI, BDI, PDI, CHDI, TODI, NDI, HXDI or any combination thereof.

8. The oral care device or component of claim 1, wherein the thermoplastic polyurethane further comprises one or more colorants, antioxidants (including phenolics, phosphites, thioesters, and/or amines), radio opacifiers, stabilizers, lubricants, inhibitors, hydrolysis stabilizers, light stabilizers, hindered amine light stabilizers, benzotriazole UV absorber, heat stabilizers, stabilizers to prevent discoloration, dyes, pigments, reinforcing agents, or any combinations thereof.

9. The oral care device or component of claim 1, wherein the oral care device or component comprises one or more of a dental aligner, a dental retainer or an orthodontic device.

10. The oral care device or component of claim 9, wherein the oral care device or component is personalized to a patient.

## Patentansprüche

1. Mundpflegevorrichtung oder -komponente, die umfasst:
eine additiv hergestellte thermoplastische Polyurethanzusammensetzung, die von (a) einer Polyisocyanatkomponente, die ein aromatisches Diisocyanat umfasst; (b) einer Kettenverlängererkomponente abgeleitet wird; wobei die Zusammensetzung frei von Polyolkomponenten mit Ausnahme der Kettenverlängererkomponente ist.

2. Mundpflegevorrichtung oder -komponente nach Anspruch 1, wobei die Kettenverlängererkomponente 1,6-Hexandiol (HDO), 1,4-Cyclohexandimethanol (CHDM) oder Kombinationen davon umfasst.

3. Mundpflegevorrichtung oder -komponente nach Anspruch 1, wobei das aromatische Diisocyanat MDI umfasst.

4. Mundpflegevorrichtung oder -komponente nach Anspruch 1, wobei das additive Herstellen eine Schmelzschichtung oder ein selektives Lasersintern umfasst.

5. Mundpflegevorrichtung oder -komponente nach Anspruch 1, wobei das thermoplastische Polyurethan biokompatibel ist.

6. Mundpflegevorrichtung oder -komponente nach Anspruch 1, wobei die Kettenverlängererkomponente von 25 Gew.-% bis 35 Gew.-% des Gesamtgewichts der Zusammensetzung beträgt.

7. Mundpflegevorrichtung oder -komponente nach Anspruch 1, wobei die Polyisocyanatkomponente ferner TDI, IPDI, LDI, BDI, PDI, CHDI, TODI, NDI, HXDI oder eine beliebige Kombination davon umfasst.

8. Mundpflegevorrichtung oder -komponente nach Anspruch 1, wobei das thermoplastische Polyurethan ferner ein oder mehrere Farbmittel, Antioxidationsmittel (einschließlich Phenole, Phosphite, Thioester und/oder Amine), Strahlungstrübungsmittel, Stabilisatoren, Gleitmittel, Inhibitoren, Hydrolysestabilisatoren, Lichtschutzmittel, sterisch gehinderte Aminlichtschutzmittel, Benzotriazol-UV-Absorber, Wärmestabilisierungsmittel, Stabilisatoren, um Verfärbung zu verhindern, Farbstoffe, Pigmente, Verstärkungsmittel oder beliebige Kombinationen davon umfasst.

9. Mundpflegevorrichtung oder -komponente nach Anspruch 1, wobei die Mundpflegevorrichtung oder-komponente einen Zahnausrichter, eine Zahnspange und/oder eine kieferorthopädische Vorrichtung umfasst.

10. Mundpflegevorrichtung oder -komponente nach Anspruch 9, wobei die Mundpflegevorrichtung oder -komponente für einen Patienten personalisiert ist.

## Revendications

1. Dispositif ou composant de soin buccal, comprenant :
une composition de polyuréthane thermoplastique fabriquée par additif dérivée (a) d'un composant polyisocyanate comprenant un diisocyanate aromatique ;
(b) d'un composant allongeur de chaîne ;
la composition étant exempte de composants polyol autres que le composant allongeur de chaîne.

2. Dispositif ou composant de soin buccal selon la revendication 1, dans lequel le composant allongeur de chaîne comprend du 1,6-hexanediol (HDO), du 1,4-cyclohexane diméthanol (CHDM) ou des combinaisons de ceux-ci.

3. Dispositif ou composant de soin buccal selon la revendication 1, dans lequel le diisocyanate aromatique comprend du MDI.

4. Dispositif ou composant de soin buccal selon la revendication 1, dans lequel la fabrication d'additif comprend une modélisation de dépôt par fusion ou un frittage sélectif au laser.

5. Dispositif ou composant de soin buccal selon la revendication 1, dans lequel le polyuréthane thermoplastique est biocompatible.

6. Dispositif ou composant de soin buccal selon la revendication 1, dans lequel le composant allongeur de chaîne est de 25 à 35 % en poids du poids total de la composition.

7. Dispositif ou composant de soin buccal selon la revendication 1, dans lequel le composant polyisocyanate comprend en outre le TDI, IPDI, LDI, BDI, PDI, CHDI, TODI, NDI, HXDI ou toute combinaison de ceux-ci.

8. Dispositif ou composant de soin buccal selon la revendication 1, dans lequel le polyuréthane thermoplastique comprend en outre un ou plusieurs colorants, des antioxydants (y compris des substances phénoliques, des phosphites, des thioesters et/ou des aminés), des radio-opacifiants, des stabilisants, des lubrifiants, des inhibiteurs, des stabilisants d'hydrolyse, des stabilisants à la lumière, des stabilisants à la lumière aux aminés à empêchement stérique, un absorbeur d'UV au benzotriazole, des stabilisants thermiques, des stabilisants pour empêcher la décoloration, des colorants, des pigments, des agents de renforcement, ou toute combinaison de ceux-ci.

9. Dispositif ou composant de soin buccal selon la revendication 1, dans lequel le dispositif ou composant de soin buccal comprend un ou plusieurs parmi un dispositif d'alignement dentaire, un dispositif de retenue dentaire ou un dispositif orthodontique.

10. Dispositif ou composant de soin buccal selon la revendication 9, dans lequel le dispositif ou composant de soin buccal est personnalisé pour un patient.
